# EUROPEAN PATENT APPLICATION

(11) **EP 2 397 088 A1**
(43) Date of publication of application: **21.12.2011**
(21) Application number: 10425209.3
(22) Date of filing: 18.06.2010
(51) Int. Cl.: A61B 17/70

(54) **Device for producing a struction which can be implanted in at least one part of the spinal column of a subject suffering from scoliosis and/or from another vertebral pathology in order to stabilise and/or correct this pathology**

(71) Applicant: Abasan S.r.l., 70121 Bari (IT)
(72) Inventor: De Giorgi, Giuseppe, 70124 Bari (BA) (IT); Gentile, Angelo, 70122 Bari (BA) (IT); Foglia, Mario Massimo, 70122 Bari (BA) (IT)
(74) Representative: Ciceri, Fabio

(57) **Abstract**

Device for producing a structure which can be implanted in at least one part of the spinal column of a subject suffering from scoliosis in order to stabilise and/or correct said condition, comprises a first rod-shaped member (1) provided with a portion (2) having a thread (3) which starts from one end (4) and extends along the longitudinal axis (X-X) of the member, towards the opposite end (5), a head (6) formed in said opposite end, said head (6) being provided with a through-opening (7) extending along an axis transverse to the longitudinal axis (X-X) of said rod-shaped member (1), a second rod-shaped member (18) positioned in the opening (7) in the head (6) of the first rod-shaped member (1), fixing means (9, 10, 12) for locking said second rod-shaped member (18) relative to the head (6) with a first segment (30) protruding, with its free end (21), from one side of said head (6) and a second segment (31) with its other free end (29) protruding from the opposite side of said head (6). The first segment (30) has a quadrilateral cross-section with two opposing sides (25, 26), one parallel to the other. The second segment (31) comprises a fork (20), the prongs of which (22, 23) have facing surfaces (27, 28), turned towards the recess (24) of the fork (20), one parallel to the other and flat, with a position parallel to the position of the planes to which the parallel opposing sides (25, 26) of the first segment (30) belong. The implantable structure comprises a plurality of devices arranged in series in which the first segment (30) of a device is positioned between the prongs (22, 23) of the fork (20) of the second segment (31) of the adjacent device.

## Description

The present invention relates to a device for producing a structure which can be implanted in at least one part of the spinal column of a subject suffering from scoliosis or plurisegmental instability or else from another pathology with degenerative instability in order to stabilise and/or correct said pathology while retaining the normal movement of flexion and extension in the sagittal plane, preferably a structure which can be implanted temporarily in at least one part of the spinal column.

The device according to the invention is preferably able to produce a structure which can be implanted on only one side of the spinal column.

As is known, scoliosis affects mainly young subjects up to skeletal maturity reached during adolescence. It consists of a helical deformity of the spinal column and manifests itself in a part of the column, sometimes even an extensive one, curved laterally relative to the vertical axis of the spinal column itself outside the median sagittal plane of the subject's body.

The lateral curvature is accompanied by rotations of the individual vertebrae as a result of which the deformity is defined as being three-dimensional, which therefore renders the pathological condition of the spinal column even more complex.

As the subject becomes older, the spinal column affected by scoliosis partly deactivates the muscular function of the area of the curvature. In the area of the chest, the deformation of the spinal column extends to the ribs which, on the concave side, flatten and, on the convex side, accentuate their curvature developing a rib hump in clinical terms.

According to the techniques currently used, the therapeutic treatment consists mainly of wearing corsets and sometimes plaster jackets which, although easy to apply on the one hand, on the other hand make it difficult for the subject to breathe. This means that the subject needs to do physical exercises daily in order to increase breathing capacity and maintain muscle tone.

There are also surgical treatments involving the use of mechanical devices for stiffening and stabilising that are fixed temporarily by intraosteal screws to the vertebrae of the spinal column in the curved area, after having straightened it.

These mechanical stiffening devices, known in the art, once they have been applied in situ on the spinal column, are associated with arthrodesis, or fusion of the bones of the vertebrae involved in the curvature. The instrumentation with screws and the arthrodesis restore the vertebral stability that the scoliosis curvature had compromised but inhibit the spinal column's natural flexion in all planes.

In the case of vertebral instability the vertebrae, especially in the thoracolumbar and lumbar areas, become clearly misaligned with respect to each other and tend to slip relatively and become dislocated axially due to pathological rotation. In these cases too the current therapeutic option consists of definitive use with arthrodesis of devices consisting of screws and rods.

The object of the present invention is therefore to create a device which can form a structure which can be implanted temporarily in a subject's spinal column, in the part of the column affected by scoliosis and/or by segmental instability, and which will overcome the problems found with known devices, or, specifically, the prevention of movement.

The object is achieved with a device in accordance with claim 1 below.

With the present invention it is possible to preserve intervertebral movement in the sagittal plane (flexion and extension) and to suppress right and left lateral flexion and axial rotation movement.

The invention will now be described in greater detail with reference to practical embodiments given by way of non-limiting example, shown on the attached drawings in which:
- Fig. 1 shows a frontal view of the first rod-shaped member of the device in a first embodiment;
- Fig. 2 shows a lateral view of the rod-shaped member of Fig. 1 rotated by 90° in relation to Fig. 1;
- Fig. 3 shows a section made along line III-III of Fig. 1;
- Fig. 4 shows a perspective view of the first rod-shaped member of the device according to the invention in a second embodiment with an orientatable and deformable semispherical annular body;
- Fig. 5 shows a perspective view of the orientatable annular body;
- Fig. 6 shows a perspective view of the second rod-shaped member of the device according to the invention;
- Fig. 7 shows a lateral view of the second rod-shaped member of Fig. 6;
- Fig. 8 shows a plan view of the same second rod-shaped member of Fig. 7;
- Fig. 9 shows the section made along line IX-IX of Fig. 7;
- Fig. 10 shows the device according to the invention with the second rod-shaped member mounted on the first rod-shaped member, the latter in its second embodiment of Fig. 4;
- Fig. 11 shows a sequence of implantable devices in a spinal column affected by scoliosis produced by the modular composition of a plurality of devices according to the invention;
- Fig. 12 shows a schematic view of a structure similar to that in Fig. 11 implanted in a part of the spinal column;
- Figs. 13, 14, 15 and 16 are schematic perspective views outlining the main stages of the fitting of the structure implanted in a part of the spinal column.

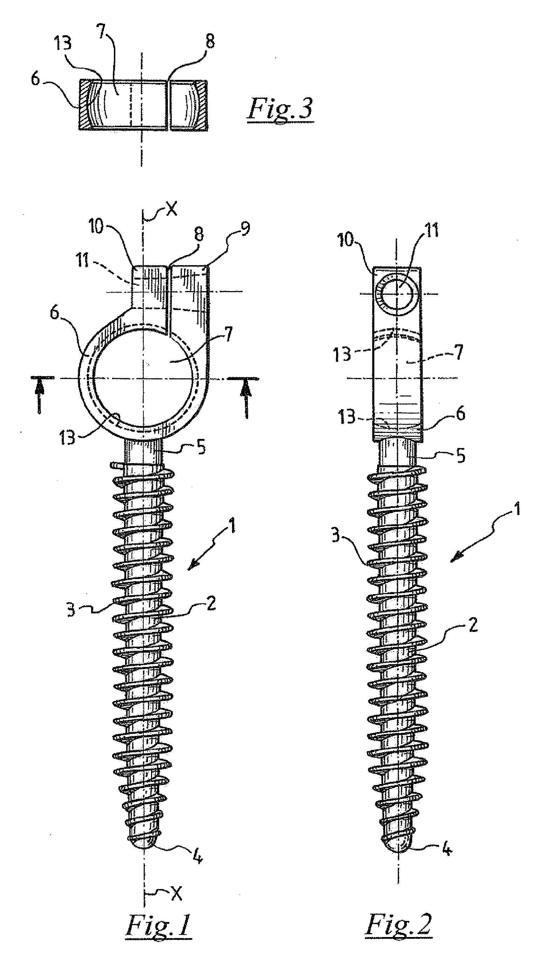

With reference to the drawings referred to above and in particular to Figs. 1, 2 and 3, the first rod-shaped member of the device is denoted in its entirety by 1. It is provided with a portion 2 having a thread 3 which extends from the extremity 4 towards the opposite extremity 5 from which a head 6 extends.

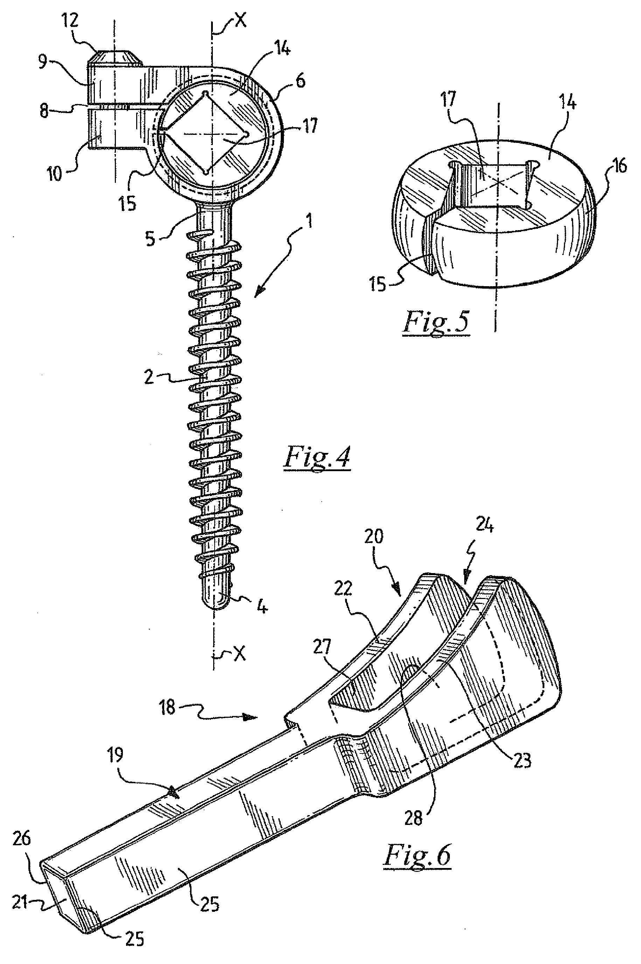

Said head has a through-opening 7 in the shape of a spherical cap having its centre on the axis of the member 1; there is a slot 8 for increasing the resilience of the head 6 and allowing clamping. At the sides of the head there are the appendages 9 and 10 with a threaded hole 11 for receiving an operating screw 12, said screw being shown in Fig. 4 in relation to the second embodiment of the first rod-shaped member.

With reference to Figs. 1, 2 and 3, it can be seen that the slot 8 of the annular head 6 is formed in a direction parallel to the longitudinal axis X-X of the rod-shaped member 1.

With reference, however, to Fig. 4 it can be seen that said slot 8 is formed in a direction perpendicular to the longitudinal axis X-X of the rod-shaped member 1 giving rise, therefore, to a second variant of the embodiment for said first rod-shaped member of the device.

With reference to Fig. 3 it can be seen that the internal wall 13 of the through-opening 7 of the annular head 6 is shaped as a spherical surface with its centre on the axis X-X of the member 1.

With reference to Fig. 4 it can be seen that, inside the opening 7 in the head 6, there is an annular body 14, which is split, and therefore made resiliently deformable, by a slot 15. Its external wall 16 is shaped as a spherical surface capable of moving together with the spherical surface of the internal wall 13 of the opening 7. This configuration brings about a form-fit such that, when the screw 12 is loosened, the body 14 can move at angles in relation to the head 6 orientating the plane of the opening 17 about the axis X-X and also transversally thereto, for reasons and in ways which will become clear from the description below.

The opening 17 is quadrilateral in shape with dimensions which can receive the second rod-shaped member of the device as can be seen from the description below. Furthermore, when the annular body 14 is positioned within the opening 7 in the head 6 of the member 1, the centre of said opening 17 is outside the axis X-X. This eccentricity allows subsequent adaptation of the accommodation of the second rod-shaped body described below.

With reference to Fig. 6 which shows the second rod-shaped member of the device according to the invention, said member is indicated in its entirety by 18. It comprises a body 19 in the shape of a parallelepiped which, at one extremity, ends with a plane 21 and, at the other end, is provided with a fork 20 formed of the parallel prongs 22 and 23 separated one from the other by a recess 24.

The prongs 22 and 23 preferably have a length of between 10 mm and 15 mm while the length of the second rod-shaped member 18 can reach a total of 35 mm.

The width of the recess 24 is preferably equal to 6 mm and, accordingly, the cross-section of the parallelepipedal body 19 is square with sides equal to 6 mm less the machining tolerances and those necessary for its smooth insertion into the recess 24 between the prongs 22 and 23. The opposite and parallel sides of body 19 are indicated by 25 and 26.

As can be seen more clearly in the description below, in the creation of an implantable structure, the parallelepipedal body 19 of a first device is arranged between the prongs 22 and 23 of the fork 20 of the rod-shaped member 18 of a second adjacent device aligned with the first.

The sides 25 and 26 are engaged slidingly between the planar surfaces 27 and 28 of the prongs with the possibility of sliding both axially along the axis Y-Y of the member 18 and transversally to said axis.

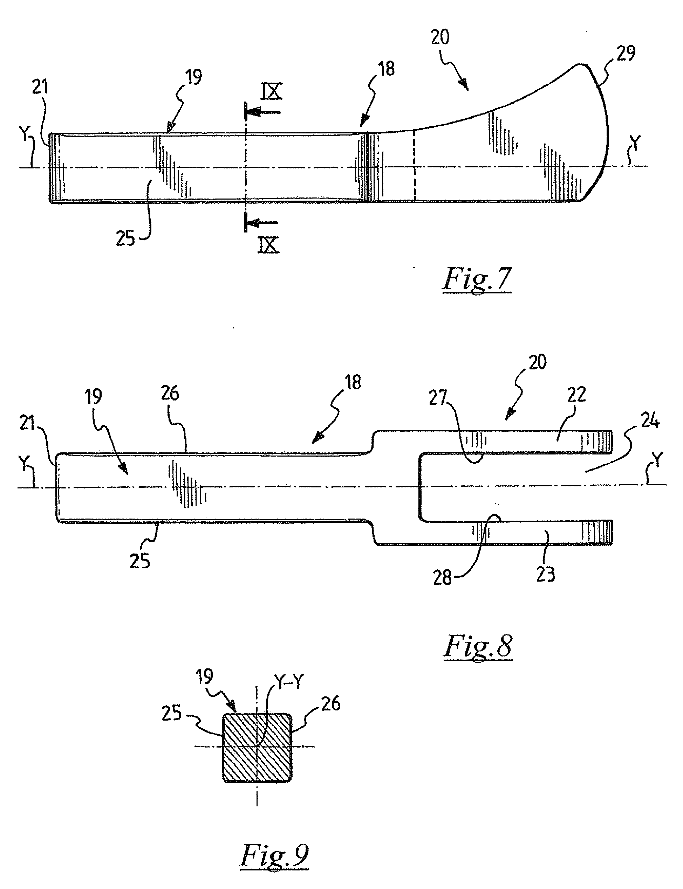

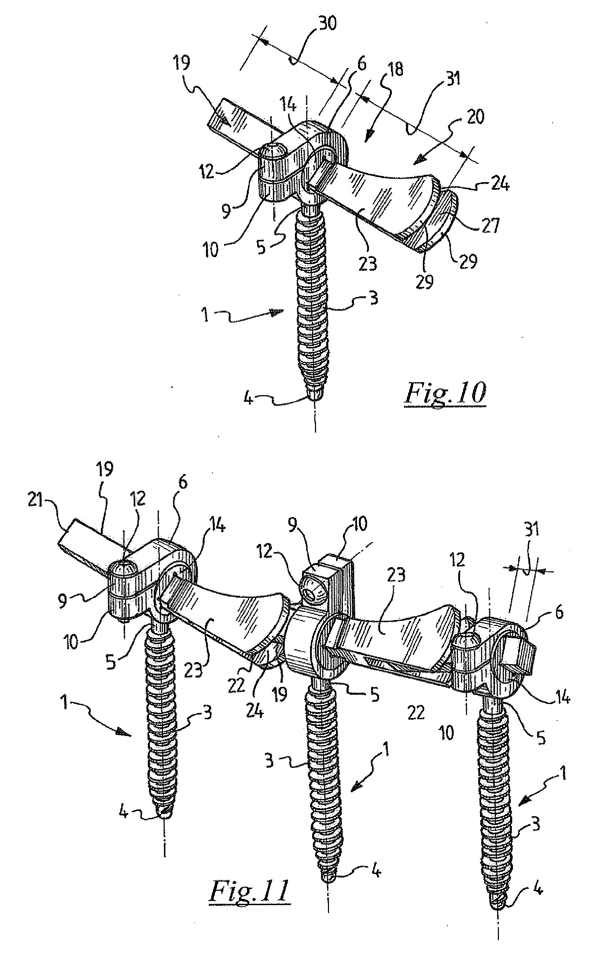

Preferably, the planar surfaces 27 and 28 of the prongs 22 and 23 not only extend in the direction parallel to the longitudinal axis Y-Y of the second rod-shaped member 18, but also widen in the direction transverse to said axis, increasing in area towards the free end 29 of the prongs. This end preferably has a convex shape, as can be seen in particular in Figs. 6 and 7. With reference to Fig. 10, the device according to the invention is formed by mounting the second rod-shaped member 18 in the head 6 of the first rod-shaped member 1 and more precisely by positioning it in the opening 17 in the orientatable annular body 14, said body being inserted into the opening 7 in the head 6 with the respective spherical surfaces being mutually engaged.

They therefore form a first segment 30 of the member 18, protruding from one side of the head 6 of the member 1 and a second segment 31, protruding from the opposite side.

An implantable structure, for the correction of scoliosis of a part of the spinal column, is produced by applying a plurality of devices according to the invention to the spinal column, arranging them one after the other and assembling them directly in the course of the implantation process.

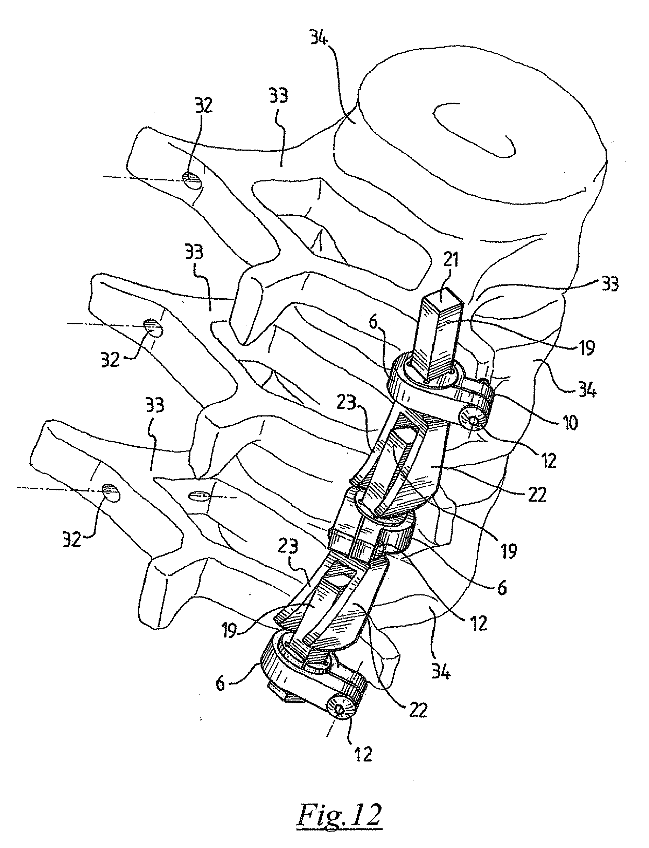

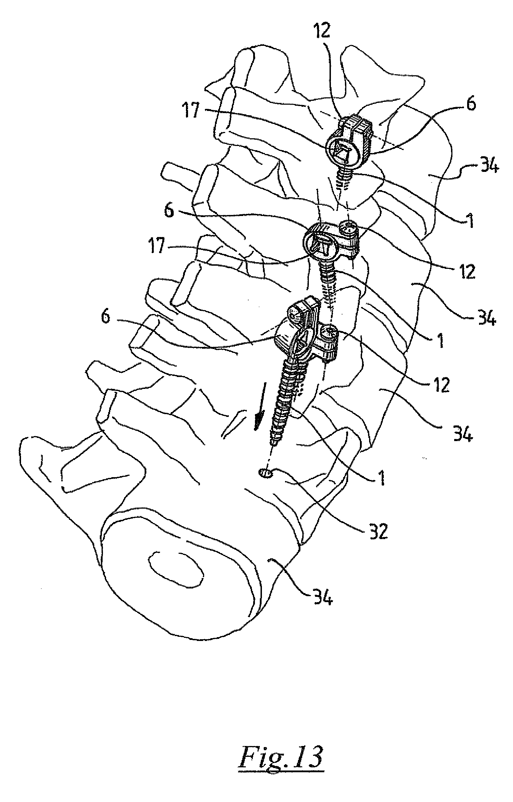

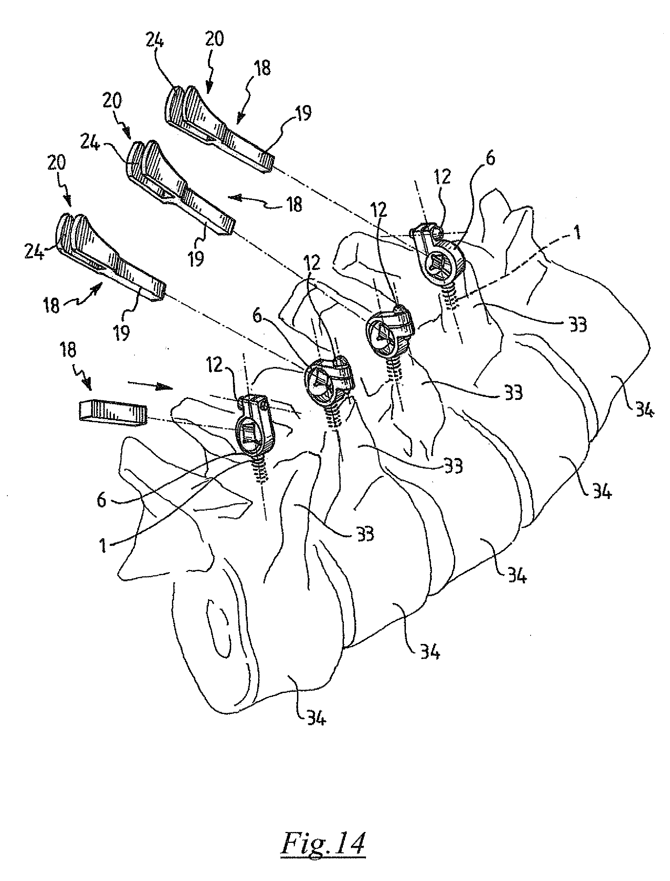

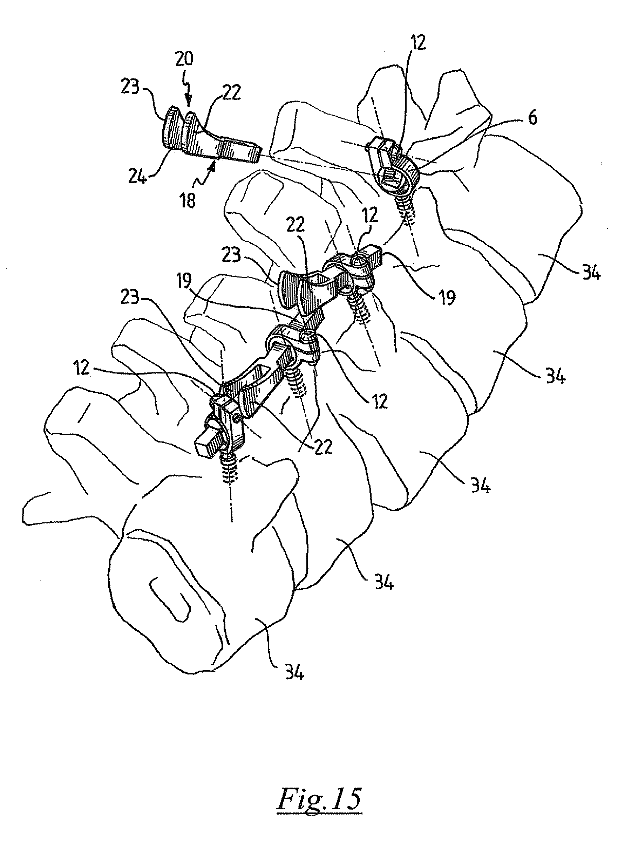

With reference to Figs. 11 to 16, after having made suitable holes 32 in the peduncles 33 from the convex side of the vertebrae 34 belonging to the part of the spinal column to be corrected, it can be seen that the structure is made by inserting with screws the rod-shaped members 1, for example three of them, as can be seen in Fig. 12, or four of them, as can be seen in Figs. 14 to 16, into respective holes 32. Once the members 1 with the respective heads 6 are in position, said heads being provided with the orientatable annular bodies 14 and aligned in planes which are practically parallel to each other, the annular bodies 14 are orientated in such a way as to make the respective openings 17 accessible from the outside of the alignment of the implanted members.

At this point the bodies 19 of the second rod-shaped members 18 are inserted in succession into the openings 17 by placing a part of the parallelepipedal body 19 of a preceding member between the prongs 22 and 23 of the second rod-shaped member of the adjacent device which follows in the alignment.

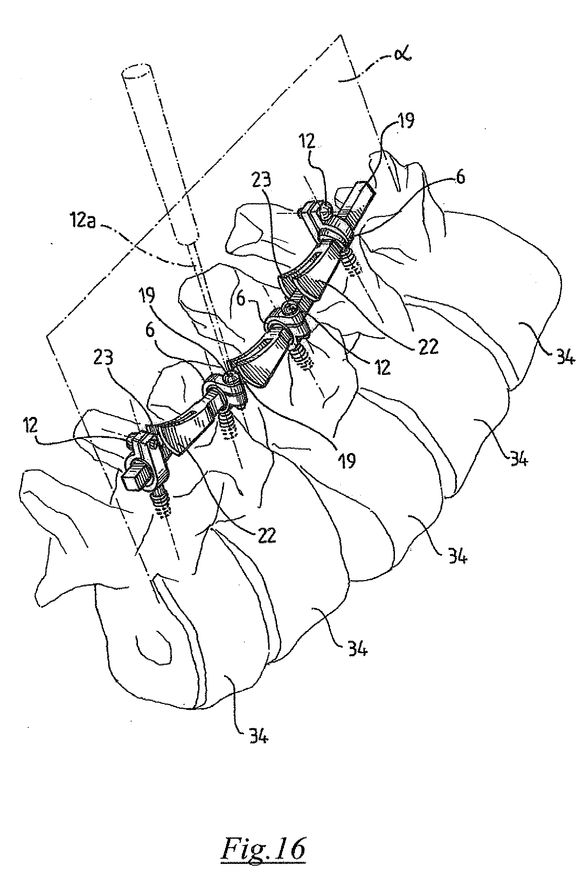

Once positioned, the parallelepipedal bodies 19, using a special tool, for example a screwdriver 12a, on the screw 12 of the clamp 9, 10, are locked in the preselected position with the planes 27 and 28 of the prongs 22 and 23 arranged in the position which allows the spinal column to move at angles, for example at an angle of between 0° and 15°, in the median sagittal plane, shown schematically by α in Fig. 16, owing to the possibility of movement allowed by the prismatic coupling between the body 19 and the planes 27 and 28 of the fork 20.

The structure, once implanted as described above, allows angular movements between the vertebrae only in the plane determined by the assembly which should be the median sagittal plane, while preventing any rotatory and lateral flexion movement because the parallelepipedal bodies 19 are free to move in the plane between the prongs 22, 23 but are prevented by said prongs from making rotatory and lateral flexion movements.

The choice of inserting rod-shaped members 1 in accordance with the first version shown in Fig. 1 or in the second version shown in Fig. 4 in the individual segments involved, is in accordance with the surgeon's discretion in relation to the local characteristics of the anatomy. Furthermore, and also preferably, when intended to form a device which, in the implantable structure is positioned at the beginning or at the end of the chain of devices, as can be seen in Figs. 11 to 16, the second rod-shaped member 18 can be used without a fork 20 with the consequence that the second segment 31 on one side of the head is in the form of a component of the same shape as the first segment, namely that of the parallelepipedal body 19.

Finally it is pointed out that the screw thread 3 in the first rod-shaped member 1 has a shape, known *per se,* suitable for non-permanent intraosteal implantation, because the structure is intended to be removed from the spinal column of the subject, once the desired corrective result has been achieved and consolidated over a number of years.

The implantable structure according to the invention, in the specific context of scoliosis, is used in cases in which treatment with corsets is ineffective and, in those involving a deformity, at the boundary between orthopaedic and surgical treatment. Another indication is that of thoracic scoliosis with slightly structured lumbar compensation while the subject is developing in which definitive instrumentation and arthrodesis are used in the thoracic part and temporary instrumentation with a structure obtained according to the invention is used, said structure acting as a guide to the correct growth of the vertebrae in the less structured lumbar curve.

The implantable structure obtained with the device according to the invention is therefore suitable for correcting vertebral misalignments in the various planes, for maintaining movement in the sagittal plane and preventing vertebral movements in the axial and frontal plane. This structure prevents the curvature and vertebral misalignments from recurring.

The invention is clearly not limited only to the embodiments described above, but in fact includes many embodiments all within the spirit and scope of the invention as claimed below.

## Claims

1. Device for producing a structure which can be implanted in a part of the spinal column of a subject suffering from scoliosis or plurisegmental instability in order to stabilise and/or correct said condition, **characterised in that** it comprises a first rod-shaped member (1) provided with a portion (2) having a thread (3) which starts at one end (4) and extends along the longitudinal axis (X-X) of the member, towards the opposite end (5), a head (6) formed in said opposite end, said head (6) being provided with a through-opening (7) extending along an axis transverse to the longitudinal axis (X-X) of said rod-shaped member (1), a second rod-shaped member (18) positioned in the opening (7) in the head (6) of the first rod-shaped member (1), fixing means (9, 10, 12) for locking said second rod-shaped member (18) relative to the head (6) with a first segment (30) protruding with its free end (21) from one side of said head (6) and a second segment (31) with its free end (29) protruding from the opposite side of said head (6).

2. Device according to claim 1, **characterised in that** said first segment (30) protruding from one side of said head (6) has a cross-section, perpendicular to its own longitudinal axis (Y-Y), that is of a quadrilateral shape with two opposite sides (25, 26) one parallel to the other, said cross-section being unchanged for at least one part of the longitudinal extension of that segment (30) next to the free end (21).

3. Device according to claim 1, **characterised in that** said second segment (31) protruding from said head (6) comprises a fork (20) formed at its free end, the prongs (22, 23) of said fork having facing surfaces (27, 28), turned towards the recess (24) of the fork, parallel to each other and flat, with a position parallel to the position of the planes to which the opposing parallel sides (25, 26) of the first segment (30) belong.

4. Device according to claim 3, **characterised in that** the planar surfaces (27, 28) of the prongs (22, 23) turned towards the recess (24) of the fork (20) extend along the axis (Y-Y) of said second segment (31), when said second member (18) is positioned in the opening (7) in the head (6) of the first member (1), from a position close to the head (6) towards the free end (29) of the segment (31) and in a transverse direction with the area of the surfaces increasing towards said free end (29).

5. Device according to claim 3, **characterised in that** the planar surfaces (27, 28) of the prongs (22, 23) turned towards the recess (24) of the fork (20) are separated one from the other by a distance of sufficient length to receive a body (19) in a form-fit and with freedom to slide both longitudinally and transversally, this body (19) having a quadrilateral cross-section with two parallel sides separated one from the other by the same distance separating the opposing parallel sides (25, 26) of said first segment (30).

6. Device according to claim 1, **characterised in that** said second segment (31) protruding from said head (6) has the same shape characteristics, both longitudinally and transversally, as said first segment (30).

7. Device according to any one of claims 1 to 6, **characterised in that** said head (6) with a through-opening (7) is shaped as a ring opened by a slot (8) and comprises a mechanism with a clamp (9, 10) with operating screws (12) placed astride said slot (8).

8. Device according to claim 7, **characterised in that** said slot (8) extends in a direction perpendicular to the longitudinal axis (X-X) of said first rod-shaped member (1) and the operating screw (12) of the clamp mechanism is positioned with its axis parallel to the longitudinal axis (X-X) of said first rod-shaped member (1).

9. Device according to claim 7, **characterised in that** said slot (8) extends in a direction parallel to the longitudinal axis (X-X) of said first rod-shaped member (1) and the operating screw (12) of the clamp mechanism is positioned with its axis perpendicular to the longitudinal axis (X-X) of said first rod-shaped member (1).

10. Device according to any one of claims 1 to 9, **characterised in that** it comprises an annular body (14) opened by a slot (15), positioned such that it is orientatable in the opening (7) in said head (6) with spherical coupling between its external surface (16) and the internal surface (13) of the opening (7) in the head (6).

11. Device according to claim 10, **characterised in that** the opening (17) in said annular body (14) is shaped like the cross-section of said first segment (30) of the second rod-shaped member (18).

12. Structure which can be implanted in a part of the spinal column of a subject suffering from scoliosis on the convex side of the curve or, in the case of plurisegmental instability, on one or both sides of the misaligned vertebrae, in order to stabilise and/or correct said condition, allowing movement in the sagittal plane and comprising a plurality of devices in accordance with one or more of claims 1 to 11 linked in series one to the other in which the first segment (30) of a device is positioned between the prongs (22, 23) of the fork (20) of the second segment (31) of the adjacent device.
